# EUROPEAN PATENT APPLICATION

(11) **EP 0 947 578 A1**
(43) Date of publication of application: **06.10.1999**
(21) Application number: 97949188.3
(22) Date of filing: 19.12.1997
(51) Int. Cl.: C11B 7/00, A23D 9/007, A23K 1/16, A23L 1/307, A61K 35/60, A61K 35/78, C07G 17/00

(54) **SUBSTANCE WITH ANTIOBESTIC AND CUMULATIVE VISCERAL FAT-REDUCING ACTIONS AND USE THEREOF**

(30) Priority: 20.12.1996 JP 35493196; 09.10.1997 JP 29343297
(71) Applicant: Janiftec, Inc., Chiyoda-ku, Tokyo 101-0051 (JP)
(72) Inventor: NADACHI, Yoshitaka Janiftec, Inc., Chuo-ku Tokyo 104 (JP); KOGA, Kenji Janiftec, Inc., Chuo-ku Tokyo 104 (JP); TANI, Masahide Janiftec, Inc., 2-9, Irifune 1-chome,Chuo-ku Tokyo 104 (JP)
(74) Representative: Baillie, Iain Cameron
(86) International application number: JP9704731
(87) International publication number: WO9828389

(57) **Abstract**

A substance with antiobese and visceral fat pad reducing functions which is derived from natural crude lipids and artificially separated therefrom and has the effect of specifically accelerating the uncoupling respiration (proton leak) of thermogenic tissue cells such as brown adipose (BA) cells of mammals including human beings, agents for medical treatment containing the above substance as the active ingredient, compositions containing the same, foods containing the above substance as the active ingredient, and feeds containing the above substance as the active ingredient, having antiobese (inhibiting an increase of visceral fat pad) and visceral fat pad reducing actions, and the above substance thus obtained specifically accelerates the uncoupling respiration (proton leak) of brown adipose (BA) cells in thermogenic tissues such as brown adipose tissues (BAT), from among vital tissue cells of mammals including human being.

## Description

### TECHNICAL FIELD

The present invention pertains to a novel substance that has the effect of specifically accelerating the in vivo uncoupling respiration (proton leak) of thermogenic tissue cells, such as brown adipose (BA) cells and the like, in thermogenic tissue, such as brown adipose tissue (BAT) and the like. In further detail, the present invention pertains to a novel substance that is derived from natural polyunsaturated fats and oils, such as fish oil and vegetable oils including linseed oil and perilla oil, etc., and that has the effect of specifically accelerating proton leak of the thermogenic tissue cells, such as brown adipose (BA) cells, etc., of the tissue cells of mammals including humans, and to compositions comprising the above-mentioned substance, and to agent having antiobese (visceral fat accumulation-inhibiting) and visceral cumulative fat-reducing functions comprising the above-mentioned substance as their effective component, use of the agent for medical treatment which has a property of reducing visceral fat pad selectively and inhibiting an increase of visceral fat pad selectively, food products that have antiobese (visceral fat accumulation-inhibiting) and visceral cumulative fat-reducing activities comprising the above-mentioned substance as their effective component, feed products that have antiobese (visceral fat accumulation-inhibiting) and visceral cumulative fat-reducing activities comprising the above-mentioned substance as their effective component, etc.

### BACKGROUND ART

A variety of studies are being performed on means of eliminating obesity in order to prevent adult disease, etc., with emphasis focused on obesity and adipose tissue.

Various new information has been reported recently relating to in vivo fat metabolism and the mechanism of in vivo fat metabolism as it relates to fat accumulation is becoming clearer on a molecular level.

Mammals, including humans, have 2 types of adipose tissues, that is, white adipose tissue (WAT) and brown adipose tissue (BAT).

White adipose (WA) cells, which are the structural components of the former white adipose tissue, have the role of storage of energy in the form of neutral fat and systemic re-supply of this neutral fat as fatty acids when necessary. These WA cells produce and secrete lipoprotein lipase that breaks down blood triglycerides in order to supply fatty acids to fat cells and muscles, or they secrete TNF-α , which inhibits the uptake of angiotensinogen that controls blood pressure and blood glucose, etc., and therefore, they are recognized as a type of secreting cell (Aihaud, G. et al.: Annu. Rev. Nutr., 12, 207-233 (1992)).

On the other hand, the latter brown adipose tissue is tissue with strong in vivo thermogenic capability, and in recent years it has become clear that this brown adipose tissue also participates in diet-induced thermogenesis when food is ingested, and deterioration of this function is very likely related to obesity (Yoshida, T.: Clinical Medicine of Obesity, Asakura Shoten, 21-31 (1993)).

However, factors involved in the metabolic activity related to a reduction in excess fats that have accumulated in vivo, that is, the metabolic activity that is a factor in their reduction, include the consumption necessary for basal metabolism in the form of activation energy of each body organ, the consumption by muscle tissue that accompanies movement, the consumption in the form of an energy source for thermogenesis that accompanies body temperature control, the consumption with the production of ketones (for instance, 3-hydroxybutyric acid, acetyl acetic acid, etc.) by intrahepatic fatty acid β-oxidation and blood secretion and excretion of the above-mentioned ketones, and the like.

The above-mentioned brown adipose tissue (BAT) is a tissue with the above-mentioned strong thermogenic activity in the form of an aggregate of brown adipose (BA) cells, and it has 300 to 400 W thermogenic activity per 1 kg tissue. The mechanism of thermogenesis by lypolysis of this brown adipose tissue has recently been clarified by biomolecular research and as a result, various drug manufacturers throughout the world are now actively pursuing developmental research of so-called antiobesity drugs that perform lypolysis by, for instance, direct stimulation of brown adipose tissue (Kawada: Rinsho to Eiyo, 85(5), 553 (1994)).

Furthermore, Friedman of Rockefeller University in the US successfully showed that when the obesity gene (ob gene) is cloned, a protein called leptin is expressed by the ob gene in white adipose cells and secreted in blood as cytokine, affecting the hypothalamic region of the brain and thereby controlling food consumption and secretion of norepinephrine (NE), a corticosteroid (J.M. Friedman et al., Nature, 372 (1 Dec.), 425-432 (1994), Newsweek, 12/14, p. 57 (1994)).

It was affirmed that when the presence of the above-mentioned norepinephrine receptor on the brown adipose cell is confirmed, the brown adipose cells are stimulated by the norepinephrine secreted from the sympathetic nerve ends via said receptor and thermogenesis is accelerated by triglyceride (TG) break down. It was concluded that the β 3 adrenergic receptor (β 3-ADR) is present in brown adipose cells and that fat metabolism is promoted by ADR agonist via this presence. This indicates that said ADR agonists increase markedly lypolysis and thermogenesis by affecting brown adipose cells.

Consequently, β 3-agonist (β 3-receptor agonist) appears to be an obesity therapeutic agent that is effective in energy consumption and its research and development is being very actively promoted. The above-mentioned antiobesity drugs have as their criterion the development of β 3-agonist, which directly affects the above-mentioned norepinephrine receptor and therefore, substances that are effective in eliminating obesity in obese model animals and that work in small animals are being developed by the US company ACC, etc., (Kawada, Rinsho to Eiyo, 85(5), 553(1994)), an agonist that is effective in humans has yet to be discovered.

In addition, with respect to diet-induced thermogenesis, Kawada, one of the present inventors, et al. were the first to clarify on a molecular level the fact that uncoupled protein (UCP) of the brown adipose cells has extremely important effects (Iwai et al., Nihon Nogei Kagakukaishi, 68(8) 1199-1205 (1994), T. Kawada et al., J. Agric. Food Chem., 39, 651-654 (1991))

Moreover, Professor Himsbegen of the University of Ottawa in Canada was the first to clarify the fact that obesity can develop without overeating using transgenic mice in which brown adipose tissue has been genetically eliminated at the 1994 International Conference on Obesity (ICO). The professor announced and focused on the fact that obesity persisted after it had developed in these mice and that this was further complicated by severe diabetes and hyperlipidemia, etc.

Moreover, Golbani et al., of the University of Ottawa announced white adipose tissue in rats was partially converted to brown adipose tissue with long-term administration of CL316,243, a specific β 3-agonist, resulting in activation of brown adipose cells and elimination of obesity. Brown adipose tissue is present in only small amounts in humans, but the above-mentioned experimental findings were the focus of attention, including the possibility of clinical use, because they indicated the possibility of partially converting white adipose tissue to brown adipose tissue with long-term administration of β 3-agonist to humans.

Furthermore, it has been clarified in recent years that protein subtypes that are very homologous and have the same functions as uncoupled protein (UCP), UCP2 and UCP3, are successively expressed to form a family. It is clear that these proteins are widely distributed not only in brown adipose cells, but also in white adipose cells, skeletal muscles, the kidneys, etc. The expression of messenger RNA (mRNA) of UCP2 is increased by a high-fat diet in experiments with mice and therefore, it is estimated that expression of the above-mentioned protein is readily affected by dietary components.

Kawada et al., are performing biochemical research relating to dietary components that induce thermogenesis of brown adipose tissue and have clarified that this activity is present in spice components (T. Kawada et al.: J. Agric. Food Chem., 39, 651-654 (1991) )), and that capsaicin, a seasoning component of red pepper, stimulates the sympathetic nerves to increase the amount of catecholamine secreted and further promote triglyceride break down (assimilation) in rats (T. Kawada, J. Nutr., 116, 1272-1278 (1986), T. Kawada, Proc. Soc. Exp. Biol. Med., 183, 250-256 (1986), T. Kawada, Biochem. Biophys. Res. Comm., 142, 259-264 (1987), T. Kawada, Agric. Biol. Chem., 51, 75-79 (1987), T. Kawada, Proc. Soc. Exp. Biol. Med., 187, 370-374 (1988), T, Kawada, Am. J. Physiol., 255, E23-E27 (1988)).

Furthermore, Iwai and Kawada et al., recently were the first to clarify the fact that allyl sulfur compounds (diallyloligosulfides), flavor components of garlic, promote secretion of norepinephrine and increase brown adipose tissue weight and accelerate triglyceride assimilation in rats on a molecular level (K. Iwai, T. Kawada et al., J. Nutr., 6, 250-255 (1995))

Moreover, research results that isolated soybean proteins and peptides activate brown adipose tissue and significantly reduce body fat in rats by accelerating fatty acid β-oxidation and promoting diet-induced thermogenesis are being reported (Saito et al., Daizo Tanpakushitsu Eiyo Kenkyukai Kaishi, 13,50-52 (1992), Komatsu et al., Daizo Tanpakushitsu Eiyo Kenkyukai Kaishi, 12, 80-84 (1991)).

In addition, Akiba et al., reports that isolated proteins with a specific structure act as a substance that controls in vivo fat accumulation, activating MFO (mixed function oxidase), such as cytochrome P-450, etc., of the hepatic drug metabolizing system in chickens and as a result, reducing blood concentrations of steroid hormones (for instance, corticosteroids) with the effect on body fat of promoting accumulation of fat tissue and thereby inhibiting in vivo fat accumulation (Akiba et al., Jpn. Poult. Sci., 31(6), 381-391 (1994), Akiba et al., Jpn. Poult. Sci., 29 (5), 287-295 (1992), Akiba et al., J. Sci. Vitaminol. 38, 247-253 (1992), Akiba et al., J. Poult. Sci., 24 (4), 220-229 (1987), Akiba et al., Poult. Sci., 63, 117-122 (1984)). Nevertheless, these effects have not been confirmed in mammals.

Moreover, it has been announced that inhibition of differentiation of precursor adipose (PA) cells to adipose cells by oral administration of peptides with a specific structure (trimer or tetramer of specific amino acids) is confirmed with 3T3-L1 cultivation systems, and in vivo fat accumulation is inhibited by administration to rats (Japanese Patent Publication No. 5-87052, Japanese Laid-Open Patent Publication No. 6-293796, Japanese Laid-Open Patent Publication No. 7-188284).

In addition, Yazawa et al., report that microorganisms that specifically produce EPA-bound phospholipids are expressed from the intestines of fish and that EPA-bound phospholipids that produce these microogranisms lower triglyceride concentrations in the liver, etc., when administered to rats (Japanese Laid-Open Patent No. 4-45751), but this is limited to specific phospholipids from special strains of EPA-producing microorganisms.

However, in general, the state of excess accumulation of body fat is defined as obesity, but it can be said that from the point of energy supply and consumption, obesity is the state where energy intake greatly exceeds consumption and is stored in large quantities as fat in the body (internal organs). Depending on the site, the in vivo accumulated fat is classified as subcutaneous fat or visceral fat and their properties are quite different. For instance, in contrast to the fact that the former rarely accumulates and is hardly metabolized, the latter readily accumulates and is easily metabolized. The reason why this visceral fat (obesity) becomes a multi-risk factor for the development of adult disease is that the fatty acids secreted from fat cells flow from the portal vein directly to the liver, accelerating insulin resistance and fat synthesis and as a result, induce sugar-resistance anomalies and high blood pressure, and as a further complication, arteriosclerosis. However, when compared to subcutaneous fat, visceral fat is metabolized quickly and therefore, increases and decreases considerably, and therefore, focusing on visceral fat metabolism anomalies, it is estimated that accumulated visceral fat can be specifically and efficiently reduced if, for instance, burning of fat in brown adipose tissue and extracorporeal elimination of this heat can be accelerated and consumption of body fat thereby promoted.

### DISCLOSURE OF THE INVENTION

As is clear from the above-mentioned prior art, the above-mentioned β 3-agonist appears to be an obesity therapeutic agent that is effective in consumption of energy, and its research and development are being very actively promoted, but an effective agonist in humans has yet to be developed, as previously explained. Moreover, information is being obtained, such as the fact that isolated soybean proteins and peptides activate brown adipose tissue and significantly reduce body fat in rats, peptides have the ability to inhibit elevation of blood triglyceride concentrations, etc., but these facts have yet to be actually used, and since, for instance, results are not obtained from amino acid mixtures with the same composition as the target peptide, efficacy is limited to only specific structures.

Moreover, specific spice components, etc., are still the only dietary components that induce thermogenesis by affecting brown adipose cells, and there are no dietary components that fall under the classification of ordinary food components with such effects. Furthermore, for instance, guaranteeing that food and drug components will have strong thermogenic activity and at the same time be very safe is an extremely important factor in their use, but from this point, there has not been sufficient basic research and there is a strong need for development of novel effective components (factors).

Therefore, finding effective components that have the ability to greatly increase lypolysis and thermogenesis by acting on thermogenic tissue, including brown adipose tissue, that is, factors that have the effect of specifically accelerating uncoupled respiration (proton leak) of thermogenic tissue cells, such as brown adipose cells, as an effective component, is an important problem.

That is, the present invention is a novel substance as the above-mentioned effective component (factor) having the effect of specifically accelerating proton leak of thermogenic tissue cells, such as brown adipose (BA) cells and the like, of the tissue cells of mammals, including humans, and compositions comprising the above-mentioned substance, and the use of the compositions as an obesity therapeutic agent having antiobese (visceral fat accumulation-inhibiting) and visceral cumulative fat-reducing functions comprising the above-mentioned substance as their effective component, food products that have antiobese (visceral fat accumulation-inhibiting) and visceral cumulative fat-reducing activities comprising the above-mentioned substance as the effective component, feed products that have antiobese (visceral fat accumulation-inhibiting) and visceral cumulative fat-reducing activities comprising said substance as the effective component, etc.

As a result of performing intense studies in order to solve the above-mentioned problem, the inventors completed the present invention upon discovering a substance (factor) with the specific activities described below:

That is, the present invention is comprised of the following technological means:
(1) A substance that is derived from crude natural lipids and artificially from said lipids, which has the function of accelerating specifically uncoupled respiration (proton leak) of thermogenic tissue cells, such as brown adipose (BA) and the like, of mammals, including humans.
(2) The substance according to above-mentioned (1), which is a substance derived from crude polyunsaturated oils and fats.
(3) The substance according to above-mentioned (2), wherein the polyunsaturated oil and fat are one selected from fish oils and vegetable oils, such as linseed oil and perilla oil and the like.
(4) The substance according to above-mentioned (1), which has the function of specifically reacting with thermogenic tissue cells, such as brown adipose cells (BA) and the like, and markedly increase the amount of uncoupled protein (UCP) and its homologues expressed in said cells.
(5) A composition comprising the substance according to above-mentioned (1).
(6) An agent for medical treatment having a property of antiobese (inhibiting an increase of visceral fat pad) and visceral fat pad reducing activities, comprising as its effective component a substance according to above-mentioned (1) with the function of specifically accelerating proton leak of thermogenic tissue cells, such as brown adipose (BA) cells and the like.
(7) A food product having antiobese (visceral fat accumulation-inhibiting) and visceral cumulative fat-reducing activities, comprising as its effective component a substance according to above-mentioned (1) with the function of specifically accelerating proton leak of thermogenic tissue cells, such as brown adipose (BA) cells and the like.
(8) A feed having antiobese (visceral fat accumulation-inhibiting) and visceral cumulative fat-reducing activities, comprising as its effective component a substance according to above-mentioned (1) with the function of specifically accelerating proton leak of thermogenic tissue cells, such as brown adipose (BA) cells and the like.

The present invention will now be explained in further detail:

The substance that is the effective component (factor) of the present invention and has the effect of accelerating specifically proton leak of thermogenic tissue cells, such as brown adipose cells, etc., is a substance present in crude natural lipids and is artificially isolated and purified from these lipids. Starting materials of the above-mentioned substance (factor) of the present invention are crude natural lipids, in particular, crude polyunsaturated fats and oils found in nature. Fish oil and natural vegetable oils such as linseed oil, perilla oil, etc., are ideal examples. However, the starting materials are not limited to these examples and any starting material can be used as needed as long as they are the same or similar to these examples. The above-mentioned substance (factor) of the present invention is a substance that is derived from the above-mentioned oils and fats and products obtained by further purification and fractionation using the above-mentioned oils and fats as the starting material can be similarly used as long as they have the same activities. In particular, semi-pure or pure products with the above-mentioned functions that have been obtained by further deoxidation, decoloration, deodorization, etc., of crude oils are used when fish oil is the starting material, and fractions with the above-mentioned activities that have been obtained by further separation of these semi-pure and pure oils can also be employed.

The above-mentioned effective component (factor) of the presence invention is used as the effective component of, for instance, drugs, food products, feed, etc., and should therefore be purified to the level needed for its specific use. Relatively pure effective component or factor can be used in processed foods, such as special health foods (functional foods, etc.), and since natural oils are used as the starting materials for feed, they can be used on the level of crude products. The above-mentioned effective component (factor) of the present invention is a substance derived from the crude natural oils and fats, such as the above-mentioned polyunsaturated fats and oils, but the substance is not edible oil or fat itself used for food, clearly because the above-mentioned effect and function of the substance are not seen with so-called ordinary oils, for example, palm oil, coconut oil, soybean oil and the like, used for food.

Taking into consideration the source and use, as well as high activity, of the above-mentioned effective component (factor) of the present invention, studies were performed using crude products and it was confirmed that these crude products have very high activity. As actually shown in the examples that are described later, as a result of performing lipid administration studies in rats, which were conducted using the above-mentioned effective component (factor) derived from fish oil, linseed oil, and perilla oil of the present invention, there was a significant reduction in amount of visceral fat among the fish oil group and there was a tendency toward a reduction with linseed oil and perilla oil groups, when compared to the lard control group.

Moreover, as a result of determining the amount of uncoupled protein (UCP) expressed in brown adipose cells of brown adipose tissue (BAT) sampled from rats, the amount expressed in the fish oil group was very significantly higher at an average of 1.6-fold and a maximum of 2-fold, and there was a tendency toward an increase in the linseed oil and perilla oil groups, when compared to the lard control group. It can be said that this high activity is indeed a surprise as it is known that that there is a tendency toward an increase in UCP in the lard group of the control, when compared to vegetable oils that are normally used in this type of experiment, etc.

Moreover, as will be actually shown in the examples given later, as a result of similarly performing experiments using substances derived from chicken egg yolk oil, salmon spawn oil, trout spawn oil, salmon milt oil, cod milt oil, scallop spermary oil, scallop ovary oil, scallop visceral oil, and oyster visceral oil, the amount of UCP expressed by these substance was high at 1.9-fold to 2.6-fold that of the control. The effective component (factor) of the present invention can be used not only as an oily substance derived from the above-mentioned marine organisms, but in any form derived from the same starting materials as needed, such fractions, purified product, processed product, extract, mixture, composition (composition and the like, as defined in the present invention).

Expression of UCP is essential if fat is to be burned among the brown adipose cells of brown adipose tissue. The essential component (factor) of the present invention has the effect of markedly increasing the amount of UCP expressed in adipose fat cells, as previously mentioned, and therefore, based on knowledge of fat metabolism on a molecular level, this underlies the fact that it is effective in controlling visceral fat accumulation.

The above-mentioned effective component (factor) of the present invention has the effect of raising the amount expressed of uncoupled protein and its homologues, which are present on the inner membrane of mitochondria (mit) of thermogenic tissue cells, such as brown adipose (BA) cells, etc., that participate in thermogenesis in mammals, including humans, and specifically accelerate uncoupled respiration of the above-mentioned cells (proton leak.). The above-mentioned substance (factor) of the present invention is a substance of unknown structure, but the above-mentioned component can be identified as a strong UCP-expressing substance that markedly increases the amount of UCP and its homologues expressed in thermogenic tissue cells, such as brown adipose cells, etc., as previously mentioned, using this expressing activity as an indicator, and separated and purified.

The above-mentioned thermogenic tissue cells such as brown adipose (BA) cells, etc., means cells of thermogenic tissue, such as brown adipose tissue, skeletal muscle tissue, immune system tissue, etc. Moreover, the above-mentioned uncoupled protein (UCP) and its homologues in the present invention are defined as substances that include UCP and protein subtypes (UCP2 and UCP3, etc.) that are thought to be homologous with UCP and have the same activity.

A visceral cumulative fat-reducing agent or visceral fat accumulation-inhibiting agent that has particularly marked anti-obesity activity can be prepared using the above-mentioned component (factor) of the present invention as the effective component. An appropriate filler, binder, extender, disintegrator, surfactant, anti-wetting agent, excipient, diluent, etc., can be used as a vehicle of the above-mentioned reducing agent or inhibiting agent in accordance with the form in which it will be used. There are no special restrictions to the form of preparation that is used and it can be selected in accordance with the purpose of use. Examples are solids, such as tablets, granules, powders, pills, capsules, etc., liquids, suspensions, emulsions, etc.

The visceral cumulative fat-reducing agent obtained in this way can be administered by an appropriate administration method and the dose can be selected as needed in accordance with the symptoms, etc., of the patient being treated. Consequently, there are no special restrictions to the dose, the administration frequency, etc.

There are no special restrictions to the administration route, administration site, etc., of each of the above-mentioned pharmaceutical preparation forms as long as the appropriate administration is selected, such as injectable administration, oral ingestion, etc.

Moreover, food products ranging from ordinary food products to specialty health food products (functional food products) can be prepared using the above-mentioned substance (factor) of the present invention as the effective component. Moreover, said substance (factor) can be used as an additive for various food products.

The types of above-mentioned food products are not limited to specialty health food products, and the present invention can also be used in food products that in themselves of course promote obesity, such as cakes, candies, chocolates, Japanese candies, meet products, fish products, prepared food products, etc.

The amount, form, etc., of the above-mentioned substance (factor) of the present invention that will be mixed in the above-mentioned food product is selected as needed in accordance with the type of food product; product concept, product form, etc. Mixing as much as 100 to 1,000 mg per one ingestion, depending on the level that can be ingested, is usually the ideal amount.

Furthermore, feed products with visceral fat accumulation-inhibiting activity and visceral cumulative fat-reducing activity for domestic animals, etc., can be prepared by mixing the above-mentioned substance (factor) of the present invention in feed.

Feed for domestic animals, such as cattle, pigs, chickens, etc., are examples of feed with which the above-mentioned substance of the present invention is mixed, and feed for domestic animals, such as beef cattle, etc., is a particularly ideal example. The amount, form, etc., of the above-mentioned substance of the present invention that is mixed in feed is selected as needed in accordance with the type of feed, rearing conditions for the domestic animal, etc. For instance, in the case of feed used to rear beef cattle, it is preferred that 50 to 500 mg be mixed in the feed.

### BRIEF DESCRIPTION OF THE DRAWINGS

Figure 1 shows an example of analysis of UCP by Western blotting.
Figure 2 shows the UCP content in brown adipose tissue (BAT) proteins.
Figure 3 shows the UCP-1 mRNA expression-promoting effects of ingestion of fish oil in rats.

### BEST MODE FOR CARRYING OUT THE INVENTION

The present invention will be explained in further detail using Examples, but the present invention is in now way limited to these examples.

### Example 1

### 1. Laboratory animals and experimental feed

Using 3-week-old Sprague-Dawley male rats, the animals were placed in groups of 5 animals/group. They were acclimated with basic feed (F-2, Funabashi Nojo Co., Ltd.) for 1 week. Rearing conditions were adjusted to a temperature of 22 to 24 °C and humidity of 50 to 60 %, with lighting being a 12-hour period (light period 8:00 to 20:00).

The experimental feed was prepared by removing soybean white expelled oil from basic feed F-2 and substituting this with lard in the lard group, fish oil (from skipjack) in the fish oil group, linseed oil in the linseed oil group and perilla oil in the perilla oil group (10 % each was added) and then molding these feed preparations into pellets. The components of the feed used in the experiments are shown in Table 1 and the fatty acid compositions of the crude oils are shown in Table 2. The main component of the lard was oleic acid, that of the fish oil was docosahexanoic acid (DHA), and that of the linseed oil and perilla oil was α-linolenic acid. The feed and water were ingested adlibitum, and the feed remnants were removed and replaced and the amount of feed ingested and body weight were determined twice a week.

**Table 1**

| Composition of experimental diets | | | | |
|---|---|---|---|---|
| | diet group | | | |
| | lard | fish oil | linseed oil | perilla oil |
| moisture(%) | 15.7 | 17.0 | 17.5 | 17.3 |
| protein(%) | 17.3 | 17.4 | 17.0 | 17.2 |
| lipid(%) | 11.0 | 11.4 | 11.3 | 11.3 |
| carbohydrate(%) | 50.5 | 49.0 | 49.0 | 48.8 |
| fiber(%) | 2.5 | 2.2 | 2.3 | 2.5 |
| ash(%) | 3.0 | 3.0 | 2.9 | 2.9 |

**Table 2**

| Fatty acid composition of dietary fats | | | | |
|---|---|---|---|---|
| | | | | (unit %) |
| fatty acid | lard | fish oil | linseed oil | perilla oil |
| C8:0 | 2.39 | | | |
| C10:0 | 1.80 | | | |
| C12:0 | 13.46 | | | |
| C14:0 | 6.38 | 3.42 | | |
| C14:1 | | 0.17 | | |
| C15:0 | | 0.94 | | |
| C15:1 | | 0.10 | | |
| C16:0 | 19.46 | 18.09 | 5.16 | 6.23 |
| C16:1 | 2.23 | 4.13 | | |
| C17:0 | 0.46 | 1.08 | | |
| C17:1 | 0.39 | 0.26 | | |
| C18:0 | 10.59 | 4.71 | 2.85 | 2.17 |
| C18:1 | 33.92 | 11.99 | 17.17 | 20.95 |
| C18:2 | 5.76 | 1.63 | 15.07 | 12.95 |
| C18:3 | 0.32 | 0.61 | 58.81 | 57.79 |
| C20:0 | 0.33 | 1.69 | 0.18 | 0.15 |
| C20:1 | 0.51 | 0.35 | | |
| C20:4 | 0.13 | 1.98 | | |
| C20:5 | | 7.16 | | |
| C22:1 | | 1.07 | | |
| C22:5 | | 1.22 | | |
| C22:6 | | 27.65 | | |
| C24:1 | | 1.63 | | |

### 2. Animal treatment method and analysis method

After rearing the animals for 9 weeks until they were 13 weeks of age, body fat was measured using a scanner for small animal body fat measurement (Nihon Clea, CL-5351 type). The animals were sacrificed by exsanguination via cardiac puncture under nembutal anesthesia and the brown adipose tissue between the shoulder blades and the adipose tissue around the kidneys and testicles were immediately isolated and weighed. Furthermore, the brown adipose tissue was placed in a poly tube and quick-frozen with liquid nitrogen and submitted to measurements of the amount of uncoupled protein (UCP), which are discussed below:

### Extraction of UCP:

BAT excised from between the shoulder blades of rats was homogenized in 300 mM sucrose (comprising 10 mM Tris-HCl with a pH of 7.5 and 2 mM EDTA) and centrifuged at 3,100 rpm and 4°C for 5 minutes in a centrifuge on a cold table. The supernatant was further centrifuged at 12,000 rpm and 4°C for 10 minutes. The precipitate that was obtained (mitochondria fraction) served as the sample.

### Electrophoresis and Western blotting

Polyacrylamide gel electrophoresis was performed with an 11 % separated gel and 50 µg sample protein per lane were submitted to analysis. The electrophoresis conditions were electrophoresis for 10 minutes at 100 V and then for 1 hour at 200 V. Transfer to film (Hybond-C, Amersham) was performed for 30 minutes at 135 mA (2.5 mA/cm²) by the semi-dry method. Film blocking was performed by immersion for 1 hour or longer in a 2 % skim milk solution. Next, the film was reacted with rabbit antibody to rat UCP (primary antibody diluted 3,000-fold) and then rinsed and further reacted with rabbit IgG-peroxidase label (secondary antibody). After rinsing, the UCP band was detected on X-ray film with a chemilluminescence detection reagent (DuPont). The concentration of the band on the film was red by the scanner and numerically represented using a computer.

### 3. Results

There was not a significant difference noted between each experimental group in terms of the increase in body weight or amount of food ingested, as shown in Table 3. Moreover, there was not a significant difference between each experimental group in terms of body fat.

When compared to the lard control group, the epididymal fat pad weight and epididymal fat pad per body weight were significantly lower in the linseed group and showed a tendency toward being lower in the fish oil group and perilla group. Moreover, when compared to the lard control group, the perirenal fat pad weight and perirenal fat pad per body weight were significantly less in the fish oil group and also showed a tendency toward being less in the linseed oil group and the perilla oil group.

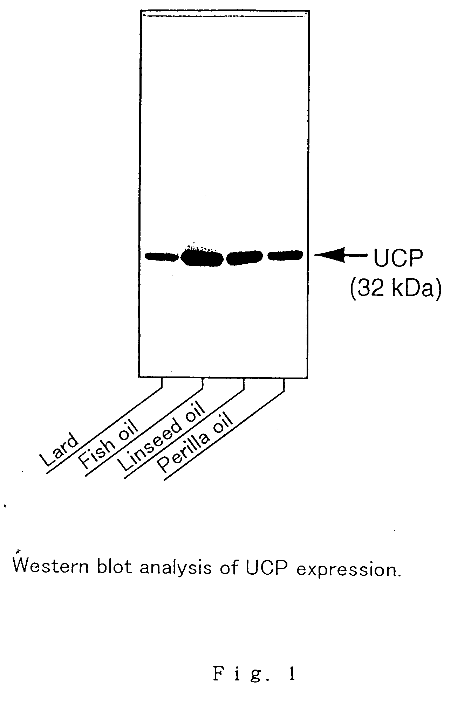

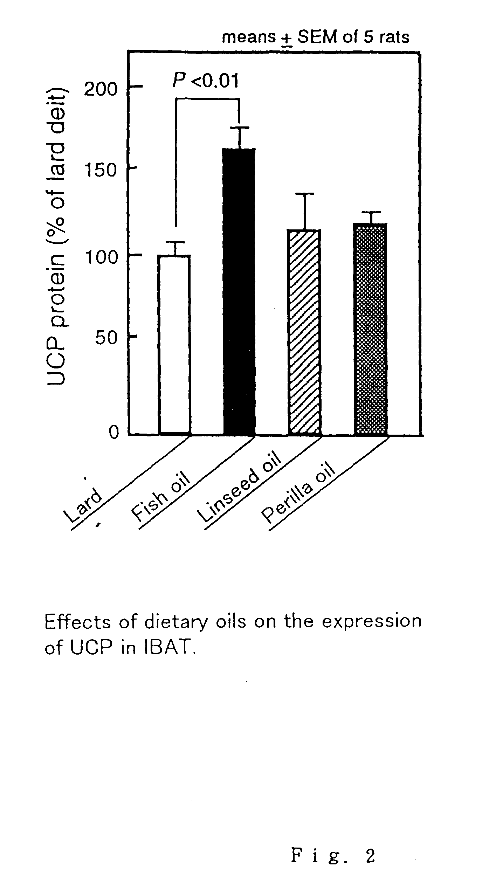

Figure 1 shows one example of analysis of rat UCP protein by Western blotting. The UCP content of BAT protein of each experimental group that was analyzed in this way is represented as a ratio with the lard control group serving as 100 in Figure 2 and Table 4.

**Table 3**

| | lard | fish oil | linseed oil | perilla oil |
|---|---|---|---|---|
| body weight gain(g) | 329.8 ± 6.1 | 326.2 ± 19.4 | 297.7 ± 33.0 | 319.7 ± 51.4 |
| total food intake of five rats(g) | 5325 | 5561 | 5248 | 5657 |
| body fat ratio (%) (average body weight(g)) | 28.90 ± 4.43 (384.9) | 28.94 ± 4.68 (382.8) | 28.85 ± 2.52 (354.9) | 31.23 ± 4.55 (374.7) |
| epididymal fat pad weight(g) | 7.71 ± 1.43 | 6.33 ± 0.91 | * 5.24 ± 0.72 | 6.76 ± 2.07 |
| perirenal fat pad weight(g) | 10.37 ± 1.93 | * 6.91 ± 2.33 | 7.91 ± 0.76 | 9.41 ± 4.05 |
| epididymal fat pad (%) per body weight | 2.00 ± 0.36 | 1.65 ± 0.15 | * 1.47 ± 0.11 | 1.77 ± 0.31 |
| perirenal fat pad (%) per body weight | 2.69 ± 0.48 | * 1.79 ± 0.52 | 2.23 ± 0.13 | 2.44 ± 0.74 Values are means ± SEM for five rats. |

| | | | | |
|---|---|---|---|---|
| * Significantly different from lard diet group at p<0.05. | | | | |

**UCP expression in IBAT**

| diet group | | | | |
|---|---|---|---|---|
| | lard | fish oil | linseed oil | perilla oil |
| means ± SEM for five rats | 100.0 ± 8.7 | 160.0 ± 13.5* | 114.7 ± 21.7 | 118.1 ± 7.8 |
| minimum value | 78.2 | 131.7 | 70.6 | 88.4 |
| maximum value | 121.0 | 195.2 | 175.7 | 131.7 |

| | | | | |
|---|---|---|---|---|
| *Significantly different from lard diet group at *p*<0.01. | | | | |

There was a significant selective reduction in visceral fat pad in the fish oil group, and a tendency toward a reduction was observed in the linseed oil and the perilla groups, when compared to the lard control group.

Moreover, as a result of measuring the amount of uncoupled protein (UCP) expressed in brown adipose cells of brown adipose tissue (BAT) collected from rats, the amount expressed in the fish oil group was very significantly higher, at 1.6-fold on the average and a maximum of 2-fold, and there was a tendency toward an increase in the linseed oil and perilla oil groups, in comparison to the control lard groups. With respect to the lard control group, it can be said that the above-mentioned high activity is indeed a surprise as it is known that that there is a tendency toward an increase in UCP with vegetable oils that are normally used in this type of experiment, etc.

Expression of UCP is essential if fat is to be burned among the brown adipose cells of brown adipose tissue. The essential component (factor) of the present invention has the effect of markedly increasing the amount of UCP expressed in adipose fat cells, as previously mentioned, and therefore, based on knowledge of fat metabolism on a molecular level, this underlies the fact that it is effective in controlling visceral fat accumulation.

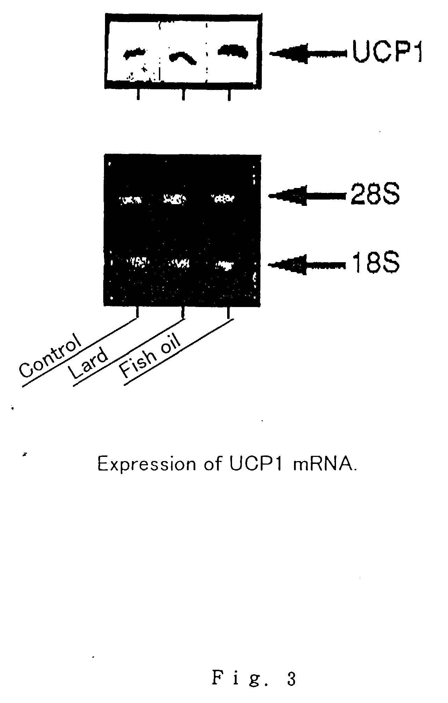

There are 3 subtypes of UCP known today, UCP-1, UCP-2, and UCP-3, and they are specifically expressed by tissue. UCP-1 is the main type expressed in brown adipose tissue. Therefore, mRNA was isolated from the mouse brown adipose tissue of mice that had ingested commercial solid feed (control group) or fish oil or lard under the same experimental conditions as used thus far and analyzed by Northern blotting using human UCP-1 cDNA as the probe. As a result, expression of UCP-1 mRNA was promoted by approximately 40 % in the fish oil group when compared to the lard-ingesting group (Figure 3). Moreover, when we consider the fact that expression of the total amount of UCP, which was analyzed by Western blotting, was promoted by 60 % or more in the fish oil-ingesting group when compared to the lard group, as previously mentioned, there is a possibility that expression of UCP mRNA by ingestion of fish oil will be accompanied by expression of other subtypes, such as UCP-1, as well as UCP-2 and UCP-3, etc.

### Example 2

### 1. Laboratory animals and experimental feed

As in above-mentioned Example 1, 4-week-old Sprague-Dawley male rats were acclimated for 1 week with basic feed and then reared for 5 weeks in the following 10 groups of 7 rats/group. The rearing environment was the same as in Example 1, but the feed was given in limited amounts and body weight was determined once a week.

The basic composition of the feed used in the experiment is shown in Table 5.

The experimental feed was 9 % of (1) lard (control group, commercial product), (2) chicken egg yolk oil (Bizen Kasei, oil from the egg yolk of chickens reared on feed containing fish oil, brand name Active DHA-0), (3) salmon spawn oil, (4) trout spawn oil, (5) salmon milt oil, (6) cod milt oil, (7) scallop spermary oil, (8) scallop ovary oil, (9) scallop visceral oil, and (10) oyster visceral oil each added to feed.

Each of above-mentioned oils (3) through (10) were prepared as follows:

That is, salmon spawn, trout spawn, salmon milt, etc., were frozen fresh. After being lyophilized, they were crushed with a mixer, ethanol was added thereto and the mixture was thoroughly shook and mixed, and the product was filtered to obtain an extract. The ethanol was removed from the extract with an evaporator to obtain an oily sample.

**Table 5**

| Composition of experimental diets | |
|---|---|
| casein(%) | 20.0 |
| cornstarch(%) | 43.8 |
| sucrose(%) | 15.0 |
| cellulose(%) | 5.0 |
| minerals(%)* | 4.0 |
| vitamins(%)* | 2.0 |
| methionine(%) | 0.2 |
| linoleic acid ethyl(%) | 1.0 |
| test oil(%) | 9.0 |

| | |
|---|---|
| *Oriental Yeast Co., Tokyo, Japan. | |

The yield from the lyophilized product of each sample and the main properties are shown in Table 6. Triglyceride determinations were performed by gas chromatography using a sample that had been dissolved in chloroform and then methylated through a silica gel column. In order to determine phospholipids, the sample was extracted by the Bligh-Dyer method and decomposed by the wet decomposition method and phosphorus was quantitatively determined by molybdenum blue absorptiometry and calculated as stearo-oleo lecithin. Fatty acid composition analysis was performed by gas chromatography after methylation in accordance with conventional methods.

**Table 6**

| | Yield^{*1} (%) | Triglyceride (%) | Phospholipid (%) | DHA^{*2} (%) | EPA^{*2} (%) |
|---|---|---|---|---|---|
| egg yolk oil | - | 59.1 | 33.8 | 5.1 | 0.8 |
| salmon spawn oil | 28.9 | 60.2 | 36.8 | 19.1 | 16.6 |
| trout spawn oil | 31.9 | 54.4 | 32.6 | 16.8 | 20.4 |
| salmon milt oil | 8.7 | 4.8 | 60.6 | 23.0 | 18.7 |
| cod milt oil | 9.5 | 6.2 | 61.5 | 14.9 | 20.3 |
| scallop spermary oil | 10.2 | 4.0 | 61.9 | 20.3 | 24.6 |
| scallop ovary oil | 7.5 | 5.4 | 61.0 | 17.1 | 30.9 |
| scallop visceral oil | 20.1 | 64.3 | 18.0 | 6.3 | 21.3 |
| oyster visceral oil | 10.7 | 70.6 | 15.7 | 5.5 | 20.8 |

| | | | | | |
|---|---|---|---|---|---|
| *1 Yield obtained from dry materials | | | | | |
| *2 The composition of fatty acid methyl esters of test oils. | | | | | |

### 2. Laboratory animal treatment method and analysis method

The laboratory animals were reared for 5 weeks and then fasted for 20 hours. Laparoscopy was performed, blood was collected from the inferior vena cava and the animals were sacrificed under ether anesthesia. Then mesenteric fat, perirenal fat pad, epididymal fat pad, and brown adipose tissue between the shoulder blades were collected and weighed and the brown adipose tissue was submitted to determination of the amount of UCP. Serum was obtained from blood by conventional methods and then serum triglycerides, cholesterol and phospholipids were measured using clinical test reagents made by Wako Junyaku Co., Ltd. The amount of UCP was determined by the method in Example 1.

### 3. Results

The determinations for each experimental group are shown in Table 7 with the control large group serving as 100. There was not a significant difference between each experimental group in terms of the increase in body weight or the amount of feed ingested.

The amount of UCP was surprisingly high in all of the experimental groups at 1.9-fold to 2.6-fold the control level. Fats in the peritoneal fats, such as mesenteric fat, etc., were significantly lower or showed a tendency toward being lower. Serum lipid concentrations, such as triglycerides, etc., similarly were significantly low or showed a tendency toward being lower when compared to the control lard group.

As in Example 1, oily substances derived from marine organisms that were used in this experiment clearly markedly increased the amount of UCP expressed in brown adipose tissue and inhibited accumulation of peritoneal fat.

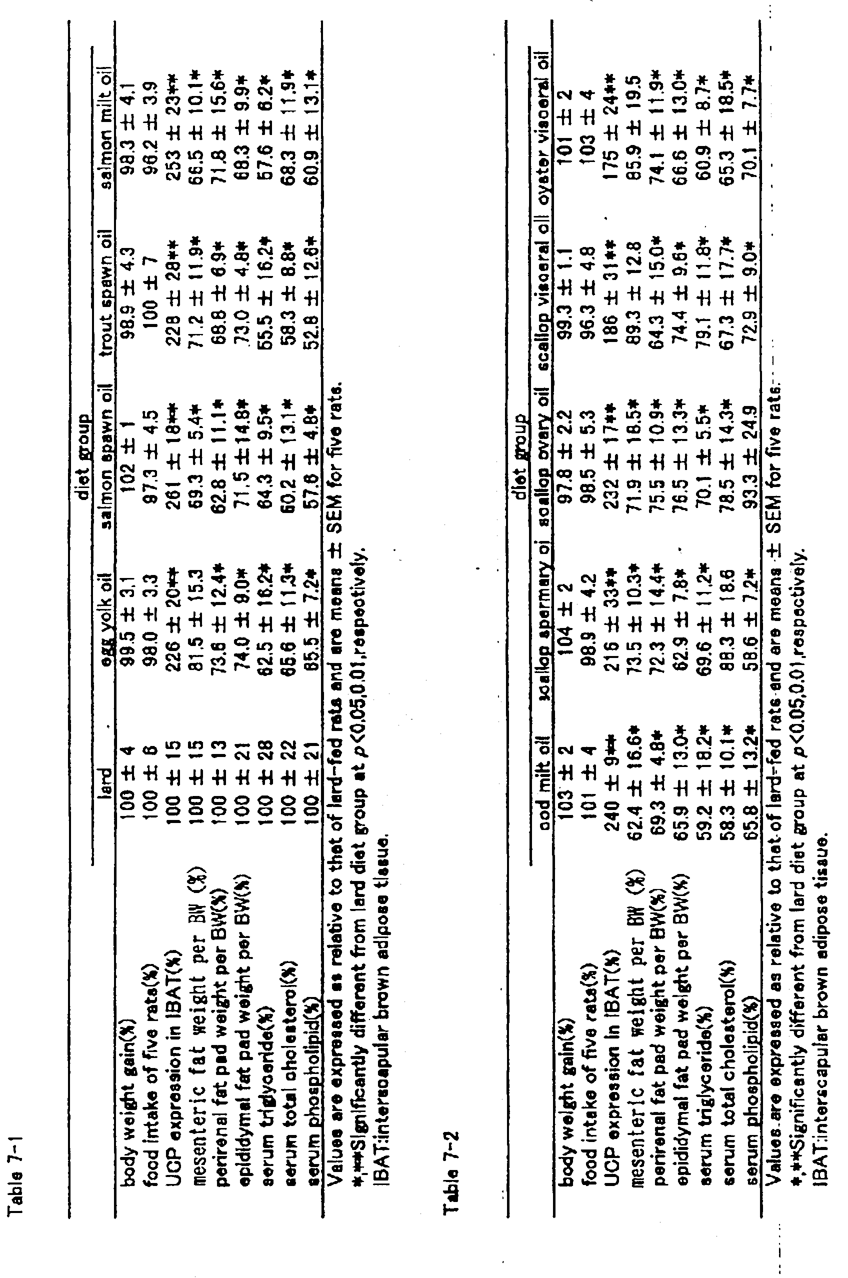

### INDUSTRIAL APPLICABILITY

Of the cells of tissues found in living beings such as mammals, including humans, the above-mentioned substance obtained by the present invention has the effect of specifically accelerating proton leak of thermogenic tissue cells such as brown adipose (BA) cells in thermogenic tissue such as brown adipose tissue (BAT). Compositions comprising this substance, antiobese (visceral fat accumulation-inhibiting) and visceral cumulative fat-reducing agents comprising this substance as their effective component, antiobese (visceral fat accumulation-inhibiting) and visceral cumulative fat-reducing food products comprising this substance as their effective component, and antiobese (visceral fat accumulation-inhibiting) and visceral cumulative fat-reducing feed comprising this substance as their effective component are presented by the present invention.

## Claims

1. A substance that is derived from crude natural lipids and artificially separated from said lipids, which has the function of specifically accelerating uncoupled respiration (proton leak) of thermogenic tissue cells, such as brown adipose cells (BA) and the like, of mammals, including humans.

2. The substance according to Claim 1, which is a substance derived from crude polyunsaturated oils and fats.

3. The substance according to Claim 2, wherein the polyunsaturated oil and fat are one selected from fish oils and vegetable oils, such as linseed oil and perilla oil and the like.

4. The substance according to Claim 1, which has the function of specifically reacting with thermogenic tissue cells, such as brown adipose cells (BA) and the like, and markedly increasing the amount of uncoupled protein (UCP) and its homologues expressed in said cells.

5. A composition comprising the substance according to Claim 1.

6. An agent for medical treatment having a property of antiobese (inhibiting an increase of visceral fat pad) and visceral fat pad reducing activities, comprising as its effective component a substance according to Claim 1 with the function of specifically accelerating proton leak of thermogenic tissue cells, such as brown adipose (BA) cells and the like.

7. A food product having antiobese (visceral fat accumulation-inhibiting) and visceral cumulative fat-reducing activities, comprising as its effective component a substance according to Claim 1 with the function of specifically accelerating proton leak of thermogenic tissue cells, such as brown adipose (BA) cells and the like.

8. A feed having antiobese (visceral fat accumulation-inhibiting) and visceral cumulative fat-reducing activities, comprising as its effective component a substance according to Claim 1 with the function of specifically accelerating proton leak of thermogenic tissue cells, such as brown adipose (BA) cells and the like.
